# EUROPEAN PATENT SPECIFICATION

(11) **EP 2 768 841 B1**
(45) Date of publication and mention of the grant of the patent: **27.09.2017**
(21) Application number: 12775237.6
(22) Date of filing: 17.10.2012
(51) Int. Cl.: C07J 43/00, A61K 31/58, A61P 35/00

(54) **ACID ADDITION SALTS OF 5ALPHA-HYDROXY-6BETA -[2-(1H-IMIDAZOL-4-YL)ETHYLAMINO]CHOLESTAN-3BETA -OL**
SÄUREADDITIONSSALZE VON 5ALPHA-HYDROXY-6BETA-[2-(1H-IMIDAZOL-4-YL)ETHYLAMINO]CHOLESTAN-3BETA-OL
SELS D'ADDITION ACIDE DE 5ALPHA-HYDROXY-6BÊTA-[2-(1H-IMIDAZOL-4-YL)ÉTHYLAMINO]CHOLESTAN-3BÊTA-OL

(30) Priority: 18.10.2011 EP 11306346
(43) Date of publication of application: 27.08.2014
(73) Proprietor: Institut National de la Santé et de la Recherche Médicale (INSERM), 75013 Paris (FR); Affichem, 31400 Toulouse (FR)
(72) Inventor: POIROT, Marc, F-31052 Toulouse (FR); POIROT, Sandrine, F-31052 Toulouse (FR); DE MEDINA, Philippe, F-31400 Toulouse (FR); PAILLASSE Michaël, F-31400 Toulouse (FR)
(74) Representative: Cabinet Plasseraud
(86) International application number: PCT/EP2012/070588
(87) International publication number: WO 2013/057148

(56) References cited:
- WO-A2-03/089449
- CN-A- 102 344 480
- DE MEDINA PHILIPPE ET AL: "Synthesis of new alkylaminooxysterols with potent cell differentiating activities: identification of leads for the treatment of cancer and neurodegenerative diseases", JOURNAL OF MEDICINAL CHEMISTRY, AMERICAN CHEMICAL SOCIETY, US, vol. 52, no. 23, 10 December 2009 (2009-12-10), pages 7765-7777, XP009131948, ISSN: 0022-2623, DOI: 10.1021/JM901063E [retrieved on 2009-09-22]
- POIROT MARC ET AL: "Novel amino-alkyl-sterols with potent differentiating activities on tumor cells", PROCEEDINGS OF THE ANNUAL MEETING OF THE AMERICAN ASSOCIATION FOR CANCER RESEARCH, AMERICAN ASSOCIATION FOR CANCER RESEARCH, US, vol. 50, 1 April 2009 (2009-04-01), page 647, XP009131954, ISSN: 0197-016X
- SILVENTE-POIROT SANDRINE ET AL: "Dendrogenin A, a promising therapy for metastatic melanoma, involving the secretion of exosomes, enriched in HSP70 and tyrosinase, and immunogenic cell death", PROCEEDINGS OF THE ANNUAL MEETING OF THE AMERICAN ASSOCIATION FOR CANCER RESEARCH, AMERICAN ASSOCIATION FOR CANCER RESEARCH, US, vol. 50, 1 April 2009 (2009-04-01), page 1130, XP009131953, ISSN: 0197-016X
- BIGHLEY L D ET AL: "Salt Forms of Drugs and Absorption", 1 January 1996 (1996-01-01), PRESERVATION OF PHARMACEUTICAL PRODUCTS TO SALT FORMS OF DRUGS AND ABSORPTION, DEKKER, UNITED STATES, PAGE(S) 453 - 499, XP008138209, ISBN: 0-8247-2812-2 page 457, paragraph 1-2 page 461, paragraph 5 - page 463, paragraph 1

## Description

The present invention relates to acid addition salts of 5α-hydroxy-6β-[2-(1*H-*imidazol-4-yl)ethylamino]cholestan-3β-ol, to their preparation and to applications thereof.

The pharmaceutically active compound 5α-hydroxy-6β-[2-(1*H*-imidazol-4-yl)ethylamino]cholestan-3β-ol is known under the name Dendrogenin A. Its structural formula is the following:

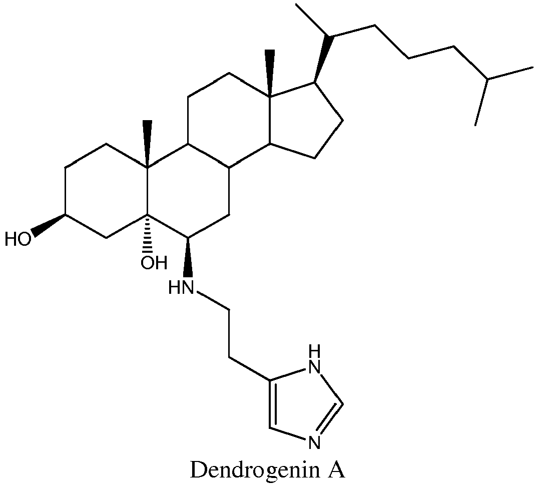

Dendrogenin A is disclosed in WO03/89449 and de Medina et al (J. Med. Chem., 2009) as free base. The solubility in water of the free base is 0.47 mg/ml.

However, the presently claimed acid addition salts of Dendrogenin A have never been disclosed up to now.

The present invention is directed to the specific acid addition salts of Dendrogenin A as claimed herein, which possess a remarkable solubility in water (up to 130 times higher than the solubility of the free base).

Due to their remarkable solubility, the salts of the present invention are expected to be more bioavailable than the free base when they are injected, thereby allowing the administration of higher doses.

The salts of the present invention are acid addition salt of 5α-hydroxy-6β-[2-(1*H*-imidazol-4-yl)ethylamino]cholestan-3β-ol formed with an acid selected from the group consisting of L-lactic acid (2(S)-hydroxypropanoic acid), malonic acid, L-malic acid, D-tartaric acid and L-tartaric acid. The salts of the invention can have a solubility in water comprised between 1 and 70 mg/ml, in particular between 1.4 and 65 mg/ml, more specifically:
- between 35 and 65 mg/ml, such as salts formed with L-lactic acid, malonic acid, L-malic acid or tartaric acid (D or L).

The salts of the invention can be prepared by the reaction of Dendrogenin A as free base with one of the above listed acids. The solvent used for the reaction may be for instance ethanol, water or toluene. It may be heated during the reaction. The resulting salt may be recovered according to methods well known by the one skilled in the art.

The present invention also relates to a pharmaceutical composition comprising a pharmaceutically acceptable carrier and one of the above mentioned acid addition salts of Dendrogenin A.

The present invention also relates to one of the above mentioned acid addition salts of Dendrogenin A for use in the treatment of neurodegenerative diseases, cancers or for activating the immune system of a patient.

The following Examples illustrate the preparation of acid addition salts of Dendrogenin A according to the present invention and other salts, not covered by the present claims, which are produced for the purposes of comparison.

### Comparative

### Example 1: 5alpha-Hydroxy-6beta-[2-(1H-imidazol-4-yl)ethylamino]cholestan-3beta-ol, hydrochloride:

Aqueous hydrochloride acid (0.9 g, 37%) is added to a solution of 5alpha-Hydroxy-6beta-[2-(1H-imidazol-4-yl)ethylamino]cholestan-3beta-ol(5.14g, 10 mmole) in ethanol (10 ml). The solution is evaporated to dryness under reduced pressure and the resulting residue is re-crystallized from methanol. The product is filtered off and re-crystallized from ethanol to afford, after filtering and drying, 5alpha-Hydroxy-6beta-[2-(1H-imidazol-4-yl)ethylamino]cholestan-3beta-ol, hydrochloride as a pale-yellow crystalline solid, having the following analytical properties: analysis found : C, 77.11; H, 10.40; N, 7.79; Cl, 6.58%. H2O, 1.75%. Calculated for C36H56ClN3O2-0.52H2O: C, 77.09; H, 10.37; N, 7.78; Cl, 6.57%. H2O, 1.73%.

### Example 2: 5alpha-Hydroxy-6beta-[2-(1H-imidazol-4-yl)ethylamino]cholestan-3beta-ol, L-tartrate:

5alpha-Hydroxy-6beta-[2-(1H-imidazol-4-yl)ethylamino]cholestan-3beta-ol (5.4 g, 10 mmol) is added to a solution of (2R,3R)2,3-dihydroxybutanedioic acid (L-(+)-tartaric acid; Fluka; 1.5 g 10 mmol) in ethanol (40 ml). The solution is evaporated to dryness under reduced pressure and the resulting residue is re-crystallized from methanol. The product is filtered off and re-crystallized from to afford, after filtering and drying, 5alpha-Hydroxy-6beta-[2-(1H-imidazol-4-yl)ethylamino]cholestan-3beta-ol, tartrate as a pale-yellow crystalline solid, having the following analytical properties: analysis found : C, 66.61; H, 9.42; N, 6.48%. H2O, 2.23%. Calculated for C36H61N3O8-0.8H2O: C, 66.60; H, 9.46; N, 6.48%. H2O, 2.22%.

### Example 3: 5alpha-Hydroxy-6beta-[2-(1H-imidazol-4-yl)ethylamino]cholestan-3beta-ol, L-malate:

5alpha-Hydroxy-6beta-[2-(1H-imidazol-4-yl)ethylamino]cholestan-3beta-ol (5.4 g, 10 mmol) is added to a solution of (2S)-(-)-hydroxybutanoic acid (L-(-)-malic acid; Fluka; 1.34 g 10 mmol) in water/Ethanol (1:1) (40 ml). The solution is evaporated to dryness under reduced pressure and the resulting residue is re-crystallized from methanol. The product is filtered off and re-crystallized from to afford, after filtering and drying, 5alpha-Hydroxy-6beta-[2-(1H-imidazol-4-yl)ethylamino]cholestan-3beta-ol, malate as a pale-yellow crystalline solid, having the following analytical properties: analysis found : C, 67.22; H, 9.53; N, 6.51%. H2O, 0.65%. Calculated for C36H61N3O7-0.23H2O: C, 67.20; H, 9.49; N, 6.53%. H2O, 0.64%.

### Comparative

### Example 4: 5alpha-Hydroxy-6beta-[2-(1H-imidazol-4-yl)ethylamino]cholestan-3beta-ol, succinate:

5alpha-Hydroxy-6beta-[2-(1H-imidazol-4-yl)ethylamino]cholestan-3beta-ol (5.4 g, 10 mmol) is added to a solution of butanedioic acid (succinic acid; Fluka; 1.18 g 10 mmol) in Ethanol (40 ml). The solution is heated to 90°C, treated with water (18g) and filtered. Upon cooling the product crystallizes and is filtered, dried to afford 5alpha-Hydroxy-6beta-[2-(1H-imidazol-4-yl)ethylamino]cholestan-3beta-ol, succinate as a pale-yellow crystalline solid, having the following analytical properties: analysis found : C, 68.99; H, 9.81; N, 6.72%. H2O, 0.78%. Calculated for C36H61N3O6-0.25H2O: C, 68.95; H, 9.74; N, 6.70%. H2O, 0.72%.

### Comparative

### Example 5: 5alpha-Hydroxy-6beta-[2-(1H-imidazol-4-yl)ethylamino]cholestan-3beta-ol, benzoate:

5alpha-Hydroxy-6beta-[2-(1H-imidazol-4-yl)ethylamino]cholestan-3beta-ol (5.4 g, 10 mmol) is added to a solution of benzoic acid (Fluka; 1.22 g 10 mmol) in toluene (40 ml). The solution is heated and filtered. Upon cooling the product crystallizes and is filtered, dried to afford 5alpha-Hydroxy-6beta-[2-(1H-imidazol-4-yl)ethylamino]cholestan-3beta-ol, benzoate as a pale-brown crystalline solid, having the following analytical properties: analysis found : C, 73.73; H, 9.66; N, 6.63%. Calculated for C39H61N3O4: C, 73.70; H, 9.61; N, 6.61%.

### Comparative

### Example 6: 5alpha-Hydroxy-6beta-[2-(1H-imidazol-4-yl)ethylamino]cholestan-3beta-ol, benzene sulfonate:

5alpha-Hydroxy-6beta-[2-(1H-imidazol-4-yl)ethylamino]cholestan-3beta-ol (5.4 g, 10 mmol) is added to a solution of benzenesulfonic acid (Fluka; 1.61 g 10 mmol) in hot toluene (40 ml). The solution is evaporated to dryness under reduced pressure and the resulting residue is re-crystallized from ethanol-ethyl acetate. The product is filtered off and dried,to afford 5alpha-Hydroxy-6beta-[2-(1H-imidazol-4-yl)ethylamino]cholestan-3beta-ol, benzenesulfonate as a pale-yellow crystalline solid, having the following analytical properties: analysis found : C, 72.80; H, 9.89; N, 5.77%. H2O, 1.11%. Calculated for C38H61N3O5S- 0.38H2O: C, 72.78; H, 9.85; N, 5.75%. H2O, 1.09%

### Comparative

### Example 7: 5alpha-Hydroxy-6beta-[2-(1H-imidazol-4-yl)ethylamino]cholestan-3beta-ol, pamoate:

A mixture of 5alpha-Hydroxy-6beta-[2-(1H-imidazol-4-yl)ethylamino]cholestan-3beta-ol (5.4 g, 10 mmol) and 4,4'methylenebis(3-hydroxy-2-naphtoic acid) (Fluka; 3.88 g 10 mmol) is heated in ethanol (40 ml). Water is added (25 ml). Upon cooling the product crystallizes and is filtered, dried to afford 5alpha-Hydroxy-6beta-[2-(1H-imidazol-4-yl)ethylamino]cholestan-3beta-ol, pamoate as a pale-yellow crystalline solid, having the following analytical properties: analysis found : C, 74.96; H, 8.15; N, 4.78%. H2O, 2.37%. Calculated for C55H71N3O8-1.15H2O: C, 74.93; H, 8.08; N, 4.77%. H2O, 2.35%

### Comparative

### Example 8: 5alpha-Hydroxy-6beta-[2-(1H-imidazol-4-yl)ethylamino]cholestan-3beta-ol, glutarate:

A solution of 1,5-pentanedioic acid (glutaric acid, Fluka, 660 mg, 5 mmole) is added to a solution of 5alpha-Hydroxy-6beta-[2-(1H-imidazol-4-yl)ethylamino]cholestan-3beta-ol(2.57 g, 5 mmole) in hot ethanol (100 ml). The solution is evaporated to dryness under reduced pressure and the resulting residue is re-crystallized from ethanol. The product is filtered off and re-crystallized from to afford, after filtering and drying, 5alpha-Hydroxy-6beta-[2-(1H-imidazol-4-yl)ethylamino]cholestan-3beta-ol, glutarate as a pale-yellow crystalline solid, having the following analytical properties: analysis found : C, 71.09; H, 10.74; N, 6.76%. H2O, 3.38%. Calculated for C37H63N3O6-1.15H2O: C, 71.07; H, 10.68; N, 6.72%. H2O, 3.31%

### Example 9: 5alpha-Hydroxy-6beta-[2-(1H-imidazol-4-yl)ethylamino]cholestan-3beta-ol, malonate:

A solution of 1,3-propanedioic acid (malonic acid, Fluka, 520 mg, 5 mmole) is added to a solution of 5alpha-Hydroxy-6beta-[2-(1H-imidazol-4-yl)ethylamino]cholestan-3beta-ol(2.57 g, 5 mmole) in hot ethanol (100 ml). The solution is evaporated to dryness under reduced pressure and the resulting residue is re-crystallized from ethanol. The product is filtered off and re-crystallized from to afford, after filtering and drying, 5alpha-Hydroxy-6beta-[2-(1H-imidazol-4-yl)ethylamino]cholestan-3beta-ol, malonate as a pale-yellow crystalline solid, having the following analytical properties: analysis found : C, 69.09; H, 9.77; N, 5.95%. H2O, 1.53%. Calculated for C35H59N3O6-0.51H2O: C, 69.05; H, 9.70; N, 5.92%. H2O, 1.51%

### Comparative

### Example 10: 5alpha-Hydroxy-6beta-[2-(1H-imidazol-4-yl)ethylamino]cholestan-3beta-ol, fumarate:

(Trans)-butenedioic acid (fumaric acid, Fluka; 1.16 g, 10 mmol) is added to a solution of 5alpha-Hydroxy-6beta-[2-(1H-imidazol-4-yl)ethylamino]cholestan-3beta-ol(5.14 g, 10 mmole) in ethanol (20ml). The solution is heated to 90°C, treated with water (18g) and filtered. Upon cooling the product crystallizes and is filtered dried to afford 5alpha-Hydroxy-6beta-[2-(1H-imidazol-4-yl)ethylamino]cholestan-3beta-ol, fumarate as a pale-yellow crystalline solid, having the following analytical properties: analysis found : C, 68.01; H, 9.58; N, 6.63%. H2O, 1.05%. Calculated for C36H59N3O6-0.36H2O: C, 67.98; H, 6.58; N, 6.61%. H2O, 1.02%.

### Comparative

### Example 11: 5alpha-Hydroxy-6beta-[2-(1H-imidazol-4-yl)ethylamino]cholestan-3beta-ol, sulfate:

A solution of sulfuric acid (Fluka, 5 ml, 1 M) in ethanol is added to a solution of 5alpha-Hydroxy-6beta-[2-(1H-imidazol-4-yl)ethylamino]cholestan-3beta-ol(2.57 g, 5 mmole) in hot ethanol (100 ml). The solution is evaporated to dryness under reduced pressure and the resulting residue is re-crystallized from ethanol. The product is filtered off and re-crystallized from ethanol to afford, after filtering and drying, 5alpha-Hydroxy-6beta-[2-(1H-imidazol-4-yl)ethylamino]cholestan-3beta-ol, sulfate as a pale-yellow crystalline solid, having the following analytical properties: analysis found : C, 62.84; H, 9.39; N, 6.94%. H2O, 5.72%. Calculated for C32H57N3O6S-1.92H2O: C, 62.81; H, 9.32; N, 6.87%. H2O, 5.65%

### Comparative

### Example 12: 5alpha-Hydroxy-6beta-[2-(1H-imidazol-4-yl)ethylamino]cholestan-3beta-ol, tosylate:

5alpha-Hydroxy-6beta-[2-(1H-imidazol-4-yl)ethylamino]cholestan-3beta-ol (5.4 g, 10 mmol) is added to a solution of 4Methylbenzenesulfonic acid (Tosylate, Fluka; g 10 mmol) in hot toluene (40 ml). The solution is evaporated to dryness under reduced pressure and the resulting residue is re-crystallized from ethanol-ethyl acetate. The product is filtered off and dried,to afford 5alpha-Hydroxy-6beta-[2-(1H-imidazol-4-yl)ethylamino]cholestan-3beta-ol, tosylate as a pale-yellow crystalline solid, having the following analytical properties: analysis found : C, 69.52; H, 9.39; N, 6.28%. H2O, 1.77%. Calculated for C39H63N305S- 0.64H2O: C, 69.49; H, 9.35; N, 6.24%. H2O, 1.71%

### Comparative

### Example 13: 5alpha-Hydroxy-6beta-[2-(1H-imidazol-4-yl)ethylamino]cholestan-3beta-ol, mesylate:

A solution of methylsulfonic acid (mesylic acid, Fluka, 5 ml, 1 M) in ethanol is added to a solution of 5alpha-Hydroxy-6beta-[2-(1H-imidazol-4-yl)ethylamino]cholestan-3beta-ol(2.57 g, 5 mmole) in hot ethanol (100 ml). The solution is evaporated to dryness under reduced pressure and the resulting residue is re-crystallized from ethanol. The product is filtered off and re-crystallized from ethanol to afford, after filtering and drying, 5alpha-Hydroxy-6beta-[2-(1H-imidazol-4-yl)ethylamino]cholestan-3beta-ol, mesylate as a pale-yellow crystalline solid, having the following analytical properties: analysis found : C, 66.10; H, 9.89; N, 7.05%. H2O, 1.63%. Calculated for C34H59N3O5S-0.53H2O: C, 66.06; H, 9.84; N, 7.01%. H2O, 1.59%

### Comparative

### Example 14: 5alpha-Hydroxy-6beta-[2-(1H-imidazol-4-yl)ethylamino]cholestan-3beta-ol, acetate:

Acetic acid (sigma,0.6g, 10 mmol) is added to a solution of 5alpha-Hydroxy-6beta-[2-(1H-imidazol-4-yl)ethylamino]cholestan-3beta-ol (5.14 g, 10 mmole) in ethanol (40 ml). The solution is evaporated to dryness under reduced pressure and the resulting residue is re-crystallized from methanol. The product is filtered off and re-crystallized from ethanol to afford, after filtering and drying, 5alpha-Hydroxy-6beta-[2-(1H-imidazol-4-yl)ethylamino]cholestan-3beta-ol, acetate as a pale-yellow crystalline solid, having the following analytical properties: analysis found : C, 71.52; H, 10.38; N, 7.41%. H2O, 0.44%. Calculated for C34H59N3O4-0.13H2O: C, 71.50; H, 10.34; N, 7.36%. H2O, 0.41%

### Comparative

### Example 15: 5alpha-Hydroxy-6beta-[2-(1H-imidazol-4-yl)ethylamino]cholestan-3beta-ol, citrate:

A mixture of 5alpha-Hydroxy-6beta-[2-(1H-imidazol-4-yl)ethylamino]cholestan-3beta-ol (5.4 g, 10 mmol) and 2-hydroxypropane-1,2,3-tricarboxylic acid (Fluka; 1.92 g 10 mmol) is heated in ethanol (40 ml). Water is added (25 ml). Upon cooling the product crystallizes and is filtered, dried to afford 5alpha-Hydroxy-6beta-[2-(1H-imidazol-4-yl)ethylamino]cholestan-3beta-ol, citrate as a pale-yellow crystalline solid, having the following analytical properties: analysis found : C, 66.06; H, 9.19; N, 6.12%. H2O, 2.11%. Calculated for C38H63N309-0.78H2O: C, 66.01; H, 9.12; N, 6.08%. H2O, 2.03%

### Example 16: 5alpha-Hydroxy-6beta-[2-(1H-imidazol-4-yl)ethylamino]cholestan-3beta-ol, L-Lactate:

A mixture of 5alpha-Hydroxy-6beta-[2-(1H-imidazol-4-yl)ethylamino]cholestan-3beta-ol (5.4 g, 10 mmol) and 2(S)-hydroxypropanoic acid (L-lactate, Fluka; 3.88 g 10 mmol) is heated in ethanol (40 ml). The solution is evaporated to dryness under reduced pressure and the resulting residue is re-crystallized from acetone-ethanol. The product is filtered off and re-crystallized from ethyl acetate to afford, after filtering and drying, 5alpha-Hydroxy-6beta-[2-(1H-imidazol-4-yl)ethylamino]cholestan-3beta-ol,lactate as a pale-yellow crystalline solid, having the following analytical properties: analysis found : C, 70.53; H, 10.01; N, 6.88%. H2O, 0.43%. Calculated for C36H61N3O5-0.14H2O: C, 70.50; H, 9.96; N, 6.85%. H2O, 0.41%

### Example 17: 5alpha-Hydroxy-6beta-[2-(1H-imidazol-4-yl)ethylamino]cholestan-3beta-ol, (D) (-) tartrate:

5alpha-Hydroxy-6beta-[2-(1H-imidazol-4-yl)ethylamino]cholestan-3beta-ol (5.4 g, 10 mmol) is added to a solution of (2S,3S)2,3-dihydroxybutanedioic acid (D-(-)-tartaric acid; Fluka; 1.5 g 10 mmol) in ethanol (40 ml). The solution is evaporated to dryness under reduced pressure and the resulting residue is re-crystallized from ethanol. The product is filtered off and re-crystallized from to afford, after filtering and drying, 5alpha-Hydroxy-6beta-[2-(1H-imidazol-4-yl)ethylamino]cholestan-3beta-ol, -(D)-tartrate as a pale-yellow crystalline solid, having the following analytical properties: analysis found : C, 67.98; H, 9.48; N, 6.63%. H2O, 4.29%. Calculated for C36H61N3O8-1.51H2O: C, 67.94; H, 9.59; N, 6.61%. H2O, 4.27%.

### Example 18-water solubility

Solubility in water at room temperature of the acid addition salts of 5alpha-Hydroxy-6beta-[2-(1H-imidazol-4-yl)ethylamino]cholestan-3beta-ol of examples 1-17 was measured according to the following method:
An adequate amount of each sample was taken. Water (50% v/v) was added to each sample, and the mixture was shaken at room temperature for 5 hours. The supernatent was filtered through a filter, and diluted with a mixed solution of water 0.1%TFA/acetonitrile as necessary to give a sample solution. The concentration (mg/ml) of the sample solution was measured by high performance liquid chromatography (HPLC) with a calibration curve method, and taken as the solubility in water at room temperature.

### HPLC analysis conditions:

HPLC purifications and analyses were carried out using an LC200 series Perkin Elmer apparatus, and diode array UV detector, using an Ultrasep C18 RP 100 column from Bischoff Chromatography.

HPLC analysis was done using an acetonitrile gradient (40% B for 8 min, then to 100% B in 20 min; A was 95:5 water/acetonitrile, 0.1% TFA; B was 95:5 acetonitrile/water 0.1% TFA) with a retention time of 18.5 min. Flow rate was 1 ml/min.

The results are listed in table 1. For comparison, the solubility of the free base is 0.47 mg/ml.

**Table 1**

| Example | Solubility in water at room temperature (mg/ml) |
|---|---|
| 1 | 23 |
| 2 | 42 |
| 3 | 46 |
| 4 | 28 |
| 5 | 31 |
| 6 | 31.5 |
| 7 | 1.4 |
| 8 | 11.2 |
| 9 | 55 |
| 10 | 16 |
| 11 | 18 |
| 12 | 24 |
| 13 | 11 |
| 14 | 4.5 |
| 15 | 7.9 |
| 16 | 65 |
| 17 | 37 |

## Claims

1. An acid addition salt of 5α-hydroxy-6β-[2-(1*H*-imidazol-4-yl)ethylamino]cholestan-3β-ol formed with an acid selected from the group consisting of 2(S)-hydroxypropanoic acid, malonic acid, L-malic acid, D-tartaric acid and L- tartaric acid.

2. A pharmaceutical composition comprising a pharmaceutically acceptable carrier and the acid addition salt according to claim 1.

3. The acid addition salt according to claim 1 for use in the treatment of neurodegenerative diseases, cancers or for activating the immune system of a patient.

## Patentansprüche

1. Säureadditionssalz von 5α-Hydroxy-6β-[2-(1*H*-imidazol-4-yl)ethyl-amino]cholestan-3β-ol, gebildet mit einer Säure, welche ausgewählt ist aus der Gruppe bestehend aus 2(S)-Hydroxypropansäure, Malonsäure, L-Äpfelsäure, D-Weinsäure und L-Weinsäure.

2. Pharmazeutische Zusammensetzung umfassend einen pharmazeutisch annehmbaren Träger und das Säureadditionssalz gemäß Anspruch 1.

3. Säureadditionssalz gemäß Anspruch 1 zur Verwendung bei der Behandlung von neurodegenerativen Erkrankungen, Krebs oder für die Aktivierung des Immunsystems eines Patienten.

## Revendications

1. Sel d'addition d'acide de 5α-hydroxy-6β-[2-(1*H*-imidazol-4-yl)éthylamino]cholestan-3β-ol formé à l'aide d'un acide choisi dans le groupe constitué de l'acide 2(S)-hydroxypropanoïque, de l'acide malonique, de l'acide L-malique, de l'acide D-tartrique et de l'acide L-tartrique.

2. Composition pharmaceutique comprenant un support pharmaceutiquement acceptable et le sel d'addition d'acide selon la revendication 1.

3. Sel d'addition d'acide selon la revendication 1 pour son utilisation dans le traitement de maladies neurodégénératives, de cancers, ou pour activer le système immunitaire d'un patient.
